Europäisches Patentamt

European Patent Office

Office européen des brevets

(11) Numéro de publication: **0 276 591 B1**

(12) **FASCICULE DE BREVET EUROPEEN**

(45) Date de publication de fascicule du brevet: **06.04.94**  (51) Int. Cl.5: **C07K 15/04**, A61K 39/21, A61K 39/42

(21) Numéro de dépôt: **87402715.4**

(22) Date de dépôt: **01.12.87**

(54) **Vaccin constitué par un vecteur viral et ADN recombinant codant notamment pour la protéine p25 du virus agent causal du S.I.D.A.**

(30) Priorité: **01.12.86 FR 8616787**

(43) Date de publication de la demande:
**03.08.88 Bulletin 88/31**

(45) Mention de la délivrance du brevet:
**06.04.94 Bulletin 94/14**

(84) Etats contractants désignés:
**AT BE CH DE ES FR GB GR IT LI LU NL SE**

(56) Documents cités:
**WO-A-86/06099**

**SCIENCE, vol. 227, 1er fevrier 1985, pp. 484-492, R. SANCHEZ-PESCADOR et al.: "Nucleotide sequence and expression of an AIDS-associated retrovirus (ARV-2)"**

**BIOTECHNOLOGY, vol. 4, no. 9, septembre 1986, pp. 790-795, N.Y., US, M.P. KIENY et al.: "AIDS virus ENV protein expressed from a recombinant vaccinia virus"**

(73) Titulaire: **TRANSGENE S.A.**
**11, rue de Molsheim**
**F-67000 Strasbourg(FR)**

Titulaire: **INSTITUT PASTEUR**
**25, rue du Docteur Roux**
**F-75015 Paris(FR)**

(72) Inventeur: **Rautmann,Guy**
**16 rue Mariano**
**F-67000 Strasbourg(FR)**
Inventeur: **Lecocq,Jean-Pierre**
**6 rue du Champ du Feu**
**F-67116 Reichstett(FR)**
Inventeur: **Kieny,Marie-Paule**
**11 rue de Gascogne**
**F-67100 Strasbourg(FR)**
Inventeur: **Gluckman,Jean-Claude**
**70 Bd. de Port Royal**
**F-75005 Paris(FR)**
Inventeur: **Montagnier,Luc**
**21 rue de Malabry**
**F-92350 Le Plessis-Robinson(FR)**
Inventeur: **Girard,Marc**
**6 rue César Franck**
**F-75015 Paris(FR)**

EP 0 276 591 B1

Rank Xerox (UK) Business Services
(3.10/3.09/3.3.3)

SCIENCE, vol. 231, 28 mars 1986, pp. 1580-1584; R.A. KRAMER et al.: HTLV-III GAG protein is processed in yeast cells by the virus POL-protease"

JOURNAL OF GENERAL VIROLOGY, vol. 67, no. 12, decembre 1986, pp. 2695-2703, SGM, GB, C. GERALD et al.: "Measles virus hea-magglutinin gene: cloning, complete nucleo-tide sequence analysis and expression in COS cells", p. 2701

Inventeur: **Salmon,Patrick**
**15 rue André Bel Sarte**
**F-75020 Paris(FR)**

(74) Mandataire: **Warcoin, Jacques et al**
**Cabinet Régimbeau**
**26, avenue Kléber**
**F-75116 Paris (FR)**

**Description**

La présente invention concerne plus particulièrement un vaccin destiné à la prévention du SIDA.

Le syndrome d'immunodéficience acquise (SIDA) est une affection virale qui présente maintenant une importance majeure en Amérique du Nord, en Europe et en Afrique centrale.

Les estimations récentes suggèrent qu'environ un million d'Américains peuvent avoir été exposés au virus du SIDA. Les individus affectés présentent une immunodépression sévère et la maladie est, en général, fatale.

La transmission de la maladie s'effectue le plus souvent par contact sexuel, bien que les personnes utilisant des stupéfiants par voie intraveineuse représentent également un groupe à haut risque ; d'autre part , un grand nombre d'individus ont été infectés par ce virus après avoir reçu du sang ou des produits sanguins contaminés.

L'agent causal de cette affection est un rétrovirus. De nombreuses affections animales ont été attribuées aux rétrovirus, mais c'est seulement récemment que des rétrovirus humains ont pu être décrits.

Alors que des rétrovirus des cellules T humaines (HTLV : human T leukemia virus) de types I et II ont été identifiés comme étant l'agent causal de certaines leucémies des cellules T chez les adultes, le rétrovirus associé à des lymphadénopathies (LAV), également appelé virus HTLV III ou AIDS-related virus (ARV) et baptisé récemment HIV1 (pour Human Immunodeficiency Virus) est généralement reconnu comme l'agent responsable du SIDA.

Le génome de plusieurs isolats de HIV a été caractérisé de façon très complète (Wain-Hobson et al., 1985 ; Ratner et al., 1985 ; Muesing et al., 1985 ; Sanchez-Pescador et al., 1985) et les similitudes de l'organisation génétique avec le virus VISNA, prototype des lentiviridae, suggèrent une parenté avec ce groupe de rétrovirus.

Les lentiviridae sont les agents de maladies persistentes à progression lente qui présentent une période d'incubation prolongée. De plus, comme le virus VISNA, le HIV montre un certain tropisme pour les tissus nerveux du cerveau.

En plus des trois gènes communément retrouvés chez tous les rétrovirus et désignés gag, pol et env, le génome du virus HIV possède au moins 4 gènes supplémentaires : Q ou sor, TAT, ART, et F ou $3'$orf (Rosen et al., 1986 ; Fisher et al., 1986).

Le gène gag code pour les protéines de structure du virion. Le messager issu de sa transcription est traduit sous la forme d'une protéine de 55.000 daltons (p55) et ce précurseur est alors maturé sous l'action d'une protéase spécifique codée par le gène pol. Le premier clivage endoprotéolytique génère une protéine de 40000 daltons (p40) et de 15000 daltons (p15) puis le deuxième clivage génère une protéine de 18000 daltons (p18) et de 25000 daltons (p25) ; l'ordre des cadres de lecture étant $H_2$N-p18-p25-p15-COOH.

L'extrémité $NH_2$-terminale de la p55 confondue avec celle de la p18 est acylée par l'acide myristique ce qui corrobore l'hypothèse selon laquelle la p18 servirait de lien entre l'acide ribonucléique génomique et la membrane dans la particule virale. La p25 est le constituant majeur de la capside du virion. Le caractère fortement basique de la p15 suggère sa liaison intime avec les acides ribonucléiques viraux (Wain-Hobson et al., 1985).

L'article de Biotechnology vol. 4, page 79 à 795, September 1986 décrit des vecteurs viraux qui comportent une partie du génome de la vaccine pour l'expression de protéines codées par le gène env du virus HIV.

La présente invention d'une manière générale a pour objet un vaccin contre le virus responsable du SIDA, caractérisé en ce qu'il est constitué par un vecteur viral qui comporte au moins :

- une partie du génome d'un virus vecteur,
- le gène gag complet ou un de ses sous-fragments, notamment un gène codant pour la protéine p25 ou un gène codant pour la protéine p18 du virus HIV, responsable du SIDA,
- ainsi que les éléments assurant l'expression de cette protéine dans les cellules eucaryotes en culture.

Dans un mode particulier de réalisation de l'invention, la partie du génome d'un virus peut être une partie du génome de poxvirus. Le poxvirus peut être le virus de la vaccine.

La séquence d'ADN codante peut être sous la dépendance d'un promoteur d'un gène du poxvirus.

Le promoteur peut être un promoteur du gène de la vaccine.

La séquence d'ADN codante peut être sous le contrôle d'un promoteur du gène de la protéine 7,5 K de la vaccine.

La séquence codante peut être clonée dans le gène TK de la vaccine.

Il est aisé de détecter des anticorps dirigés contre les protéines codées par le gène gag dans les sérums des malades atteints de SIDA, à l'exception de la protéine p15. Cette réponse immunitaire révèle le caractère fortement immunogène de ces différentes protéines chez les sujets infectés par le virus HIV. On

EP 0 276 591 B1

remarque également que lorsqu'un individu est séropositif, la majorité de ses anticorps est dirigée contre la p25 et il faut attendre la phase aiguë de la maladie pour observer un effondrement du titre des anticorps anti-p25. Cette observation établissant un parallèle entre la réponse immunitaire et l'évolution de la pathologie désigne la p25 comme une des cibles contre lesquelles il faut dresser une réponse immunitaire pour obtenir une protection contre l'issue fatale de la maladie.

Plusieurs publications soulignent l'importance des protéines de structure des virions dans l'induction des mécanismes d'immunité de type cellulaire. Parmi ces travaux il faut citer le cas du virus Influenza pour lequel une réaction CTL (lymphocytes T cytotoxiques) dirigée contre la nucléoprotéine (composante majeure de la capside du virion) d'un sous-type viral est capable de protéger également contre un autre sous-type alors qu'il n'existe pas de réaction immunitaire croisée entre sous-types pour les anticorps induits par les glycoprotéines de surface (hémagglutinine et neuraminidase). La structure primaire de ces glycoprotéines présente des zones variables dont les séquences peptidiques sont spécifiques à chaque sous-type. C'est cette divergence qui est responsable de l'absence de réaction immunitaire croisée. Une telle divergence existe aussi entre les différents isolats du HIV, elle est maximale pour les séquences de la glycoprotéine ENV (jusqu'à 35 %) mais minimale (3 à 5 %) pour celles du gène gag (Starcick et al., 1986). C'est pourquoi l'utilisation de la protéine p25 codée par le gène gag pour stimuler une résponse immunitaire contre l'infection par le HIV est une stratégie adoptée dans la présente invention.

Dans son aspect préféré, la présente invention concerne en effet l'expression de la protéine p25 en utilisant le virus de la vaccine comme vecteur. Des développements techniques récents ont autorisé la manipulation du génome du virus de la vaccine et son utilisation comme vecteur d'expression de gènes clonés. Des virus recombinants vivants ont permis d'exprimer des antigènes étrangers et même d'obtenir des immunisations contre différentes maladies virales (Herpes, Influenza, Hépatite B etc) ou parasitaires (Malaria) (M. Mackett et al., 1986 ; B. Moss, 1985).

L'expression d'une séquence codant pour une protéine étrangère par le virus de la vaccine (VV) implique nécessairement deux étapes :

1) la séquence codante doit être alignée avec un promoteur de VV et être insérée dans un segment non essentiel du génome de VV, cloné dans un plasmide bactérien approprié ;

2) les séquences d'ADN de VV situées de part et d'autre de la séquence codante doivent permettre des recombinaisons homologues entre le plasmide et le génome viral ; une double recombinaison réciproque conduit à un transfert de l'insert d'ADN du plasmide dans le génome viral dans lequel il est propagé et exprimé (Panicali et Paoletti, 1982 ; Mackett et al., 1982 Smith et al., 1983 ; Panicali et al., 1983).

Bien entendu, l'utilisation de ce type de vecteur implique souvent une délétion partielle du génome du virus vecteur.

La présente invention concerne plus particulièrement un vaccin caractérisé en ce que le vecteur viral comporte au moins :

- une partie du génome d'un virus vecteur,
- un gène codant pour la protéine p25 du virus HIV, responsable du SIDA,
- ainsi que les éléments assurant l'expression de cette protéine dans des cellules.

Par virus responsable du SIDA, on entend notamment désigner le virus HIV de même que d'éventuels mutants ponctuels ou des délétions partielles de ces virus, ainsi que les virus apparentés.

Les vecteurs viraux, dans la partie correspondant au génome du virus vecteur (distinct du virus responsable du SIDA), peuvent être constitués à partir du génome d'un virus d'origine quelconque. Toutefois, on préfèrera utiliser une partie du génome d'un poxvirus et plus particulièrement une partie du génome de la vaccine.

Les conditions nécessaires pour l'expression d'une protéine hétérologue dans le virus de la vaccine ont été rappelées précédemment.

De façon générale, le gène en cause, par exemple le gène p25, devra, pour pouvoir être exprimé, être sous la dépendance d'un promoteur d'un gène de la vaccine ; ce promoteur sera en général le promoteur de la protéine 7,5K. En outre, la séquence codante devra être clonée dans un gène non essentiel de la vaccine qui pourra éventuellement servir de gène marqueur. Dans la plupart des cas, il s'agira du gène TK.

Les techniques mises en oeuvre pour l'obtention de la protéine p25, les cultures cellulaires et les techniques de vaccination sont identiques à celles qui sont pratiquées actuellement avec les vaccins connus et ne seront pas décrites en détail.

Les exemples ci-après permettront de mettre en évidence d'autres caractéristiques et avantages de la présente invention.

Les figures ci-annexées sont les suivantes :

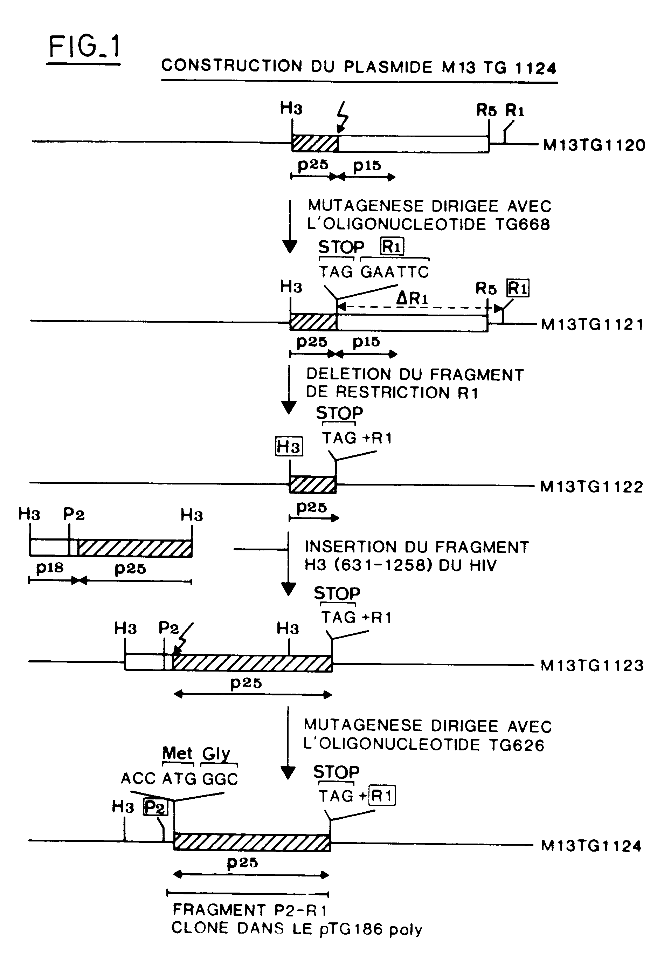

. Figure 1 : construction du plasmide M13TG1124,

4

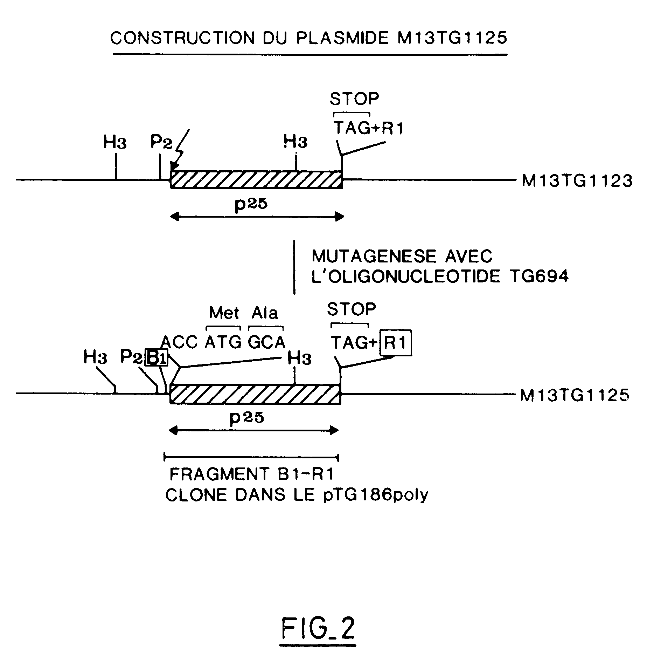

. Figure 2 : construction du plasmide M13TG1125,
les abréviations utilisées sont les suivantes :

p15     cadre de lecture de la protéine p15 du HIV,

p18     cadre de lecture de la protéine p18 du HIV,

p25     cadre de lecture de la protéine p25 du HIV,

séquences nucléotidiques provenant du HIV,

cadre de lecture de la protéine p25 du HIV

localisation de la mutagénèse dirigée,

Δ fgt. R1     délétion du fragment de restriction EcoRI,

mise en évidence des sites de restriction utilisés,

H3,R1,R5,B1,P2     enzymes de restriction, respectivement : HindIII, EcoRI, EcoRV, BamHI, PvuII,

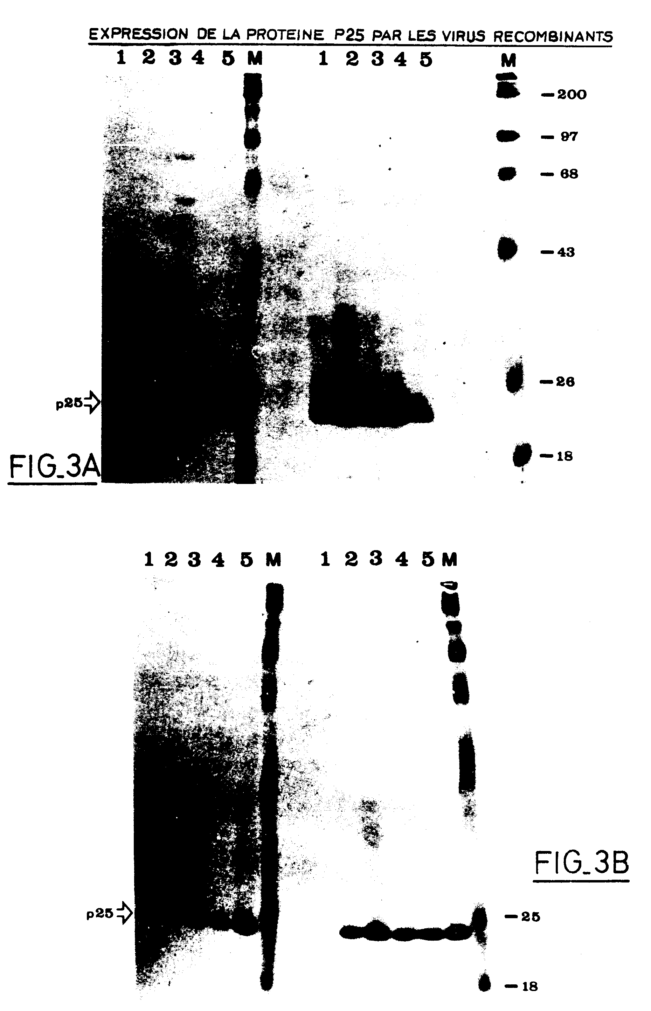

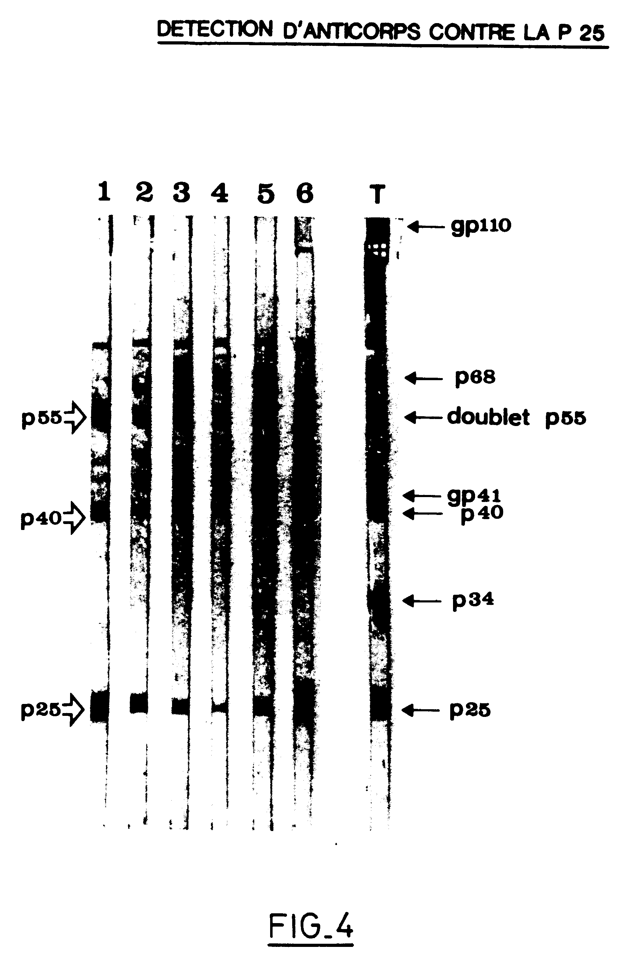

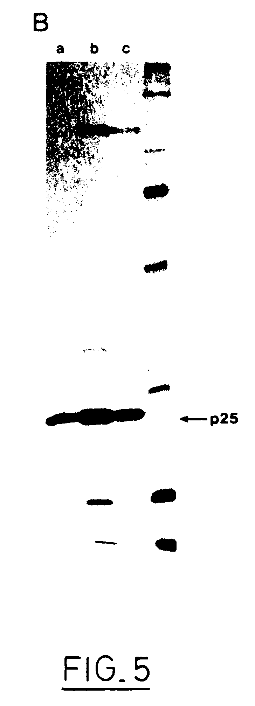

. Figure 3 : autoradiographie montrant la production de protéine p25 par les virus recombinants (3A et 3B),
. Figure 4 : détection d'anticorps contre p25,
. Figure 5 : immunoprécipitation de la p25.

Exemple 1 Construction du plasmide M13TG1123.

La protéine p25 dérive d'un précurseur polyprotéique p55 ; sa séquence correspondante en acide nucléique est donc dépourvue des signaux d'initiation et de terminaison de traduction aux extrémités du cadre de lecture. Deux mutagénèses dirigées sont effectuées, l'une pour introduire un ATG en amont, l'autre pour placer un codon "STOP" en aval de la séquence codante.

. Clonage et mutagénèse de la séquence correspondant à la portion COOH terminale de la p25.

Le fragment de restriction HindIII (coordonnée 1258 dans Wain-Hobson et al., 1985) EcoRV (coordonnée 2523) du plasmide pJ19.13 (Wain-Hobson et al., 1985) a été cloné dans les sites correspondants du vecteur M13TG131 (Kieny et al., 1983) pour former le plasmide M13TG1120.
L'arrêt de la traduction à l'extrémité du cadre de lecture de la p25 va être provoqué par l'introduction d'un codon "STOP" en phase, au nucléotide de coordonnée 1425. L'oligonucléotide de synthèse TG668 5'-GGCTCATGAATTCCTACAAAACTC-3' a été utilisé pour réaliser cette mutagénèse sur le plasmide M13TG1120. Le criblage des clones obtenus après la mutagénèse dirigée, réalisé avec l'oligonucléotide TG668 marqué avec l'isotope $^{32}$P, a permis d'isoler le plasmide M13TG1121 (Figure 1). Le petit fragment de restriction EcoRI de ce plasmide a été délété pour créer le plasmide M13TG1122.

. Clonage et mutagénèse de la séquence correspondant à la portion $NH_2$-terminale de la p25.

La fragment de restriction HindIII (coordonnées 631-1258 dans Wain-Hobson et al., 1985) du plasmide pJ19-17 a été cloné dans le site HindIII du plasmide M13TG1122 pour former le plasmide M13TG1123. L'initiation de la traduction à l'extrémité $NH_2$-terminale du cadre de lecture de la p25 est réalisée par l'addition d'un codon initiateur en amont du nucléotide de coordonnée 732. L'oligonucléotide de synthèse TG626 5′-CACTATAGGGCCCATGGTGCTGACC-3′ utilisé pour cette mutagénèse à été conçu pour obtenir également la mutation des séquences au voisinage de l'ATG initiateur, conformément aux règles de Kozak (Kozak M., 1986). Le criblage des clones obtenus après la mutagénèse dirigée, réalisé avec l'oligonucléotide TG626 marqué avec l'isotope $^{32}P$ a permis d'isoler le plasmide M13TG1124. Celui-ci renferme un cadre de lecture qui code pour une protéine p25 allongée à son extrémité $NH_2$-terminalede deux acides aminés supplémentaires : une méthionine suivie d'une glycine. Le reste de la séquence protéique est rigoureusement identique à celle de la p25 du virus HIV.

Exemple 2 Construction du plasmide M13TG1125.

Ce plasmide ne diffère du plasmide M13TG1123 que par la substitution du deuxième codon glycine par le codon alanine à l'extrémité $NH_2$-terminale de la protéine. Les étapes de clonage et de mutagénèse de la partie COOH terminale ainsi que le clonage de la partie $NH_2$-terminale sont identiques à celles décrites dans l'exemple 1.

. Mutagénèse de la partie $NH_2$-terminale.

L'oligonucléotide de synthèse TG694 5′-GCACTATAGGTGCCATGGTGCTGACCTGGATCCTGTGTCC- 3′ a été utilisé pour introduire un codon méthionine suivi d'un codon alanine en amont du premier codon proline de la p25 du virus HIV. De plus un site pour l'enzyme de restriction BamHI a été placé 17 nucléotides en amont de l'ATG, lors de la mutagénèse du plasmide M13TG1123. Le criblage des clones par l'oligonucléotide TG694 marqué avec l'isotope radioactif $^{32}P$ a permis d'isoler le plasmide M13TG1125 (voir figure 2).

Exemple 3 Construction du plasmide pTG2101

Pour obtenir l'expression de la p25 par le vecteur vaccine, la séquence codante de cette protéine a été placée sous le contrôle du promoteur du gène 7,5K du virus de la vaccine.
Le plasmide M13TG1124 a été coupé par les enzymes de restriction PvuII et EcoRI et le fragment d'ADN codant pour la p25 a été introduit dans le plasmide pTG186POLY (brevet français 86.05043) successivement traité par l'enzyme BamHI, la DNA polymérase et l'enzyme EcoRI. Les clones obtenus, après l'action de la T4 DNA ligase et transfection dans des bactéries, sont analysés par cartographie de restriction ; le plasmide pTG2101 a été sélectionné.

Exemple 4 Construction du plasmide pTG2102.

La séquence p25 du plasmide M13TG1125 a également été placée sous le contrôle du promoteur du gène 7,5K du virus de la vaccine.
Les plasmides M13TG1125 et pTG186POLY ont été coupés par les enzymes de restriction BamHI et EcoRI. Les produits de ces réactions ont été mélangés, soumis à l'action de la T4 DNA ligase et transfectés dans des bactéries. Les clones obtenus ont été analysés par cartographie de restriction et le plasmide pTG2102 a été retenu.

Exemple 5 Introduction du gène p25 dans le génome du virus de la vaccine et isolement de virus recombinés.

La stratégie décrite par Smith et al. (1983) repose sur la recombinaison génétique qui se réalise in vivo entre les séquences homologues des génomes du virus de la vaccine, dans la cellule infectée. Ce phénomène permet le transfert d'un fragment d'ADN cloné dans un plasmide vers le génome viral. L'utilisation du gène de la thymidine kinase (TK) du virus de la vaccine permet non seulement de provoquer l'intégration de l'ADN étranger dans le locus de ce gène mais aussi de disposer d'un marqueur phénotypique pour la sélection des virus recombinants qui sont devenus $TK^-$.

6

Les virus TK⁻ peuvent être sélectionnés par étalement sur une lignée cellulaire TK⁻, en présence de 5-bromo-déoxyuridine (5BUDR) (Mackett et al., 1982). Un virus TK⁻ peut y répliquer son ADN normalement et former des plages visibles.

Le virus de la vaccine se propage dans le cytoplasme des cellules infectées plutôt que dans leur noyau. C'est pourquoi il n'est pas possible de tirer avantage de la machinerie de réplication et de transcription de l'ADN de l'hôte et il est nécessaire que le virion possède les composants pour l'expression de son génome. L'ADN de VV purifié est non infectieux.

Afin de générer les recombinants, il est nécessaire d'effectuer simultanément l'infection cellulaire avec du virion VV et une transfection avec le segment d'ADN étranger cloné qui porte une zone d'homologie avec l'ADN de la vaccine. Toutefois, la génération des recombinants est limitée à la petite proportion des cellules qui sont compétentes pour la transfection par l'ADN.

L'utilisation comme virus infectieux vivant d'un mutant thermosensible (ts) de la vaccine qui est incapable de se propager à une température non permissive de 39,5°C (Drillien et Spehner, 1983) diminue le bruit de fond constitué par les virus non-recombinants. Lorsque les cellules sont infectées par un mutant ts dans des conditions non permissives et transfectées avec l'ADN d'un virus de type sauvage, la multiplication virale interviendra seulement dans les cellules qui sont compétentes pour la transfection et dans lesquelles une recombinaison entre l'ADN viral sauvage et le génome du virus ts aura eu lieu; aucun virus ne se multipliera dans les autres cellules, en dépit du fait qu'elles ont été infectées. Si un plasmide recombinant contenant un fragment de l'ADN de vaccine tel que le pTG2101 ou le pTG2102 est inclus dans le mélange de transfection, à la concentration appropriée, avec l'ADN du type sauvage, il est également possible d'obtenir qu'il participe à la recombinaison homologue avec l'ADN de la vaccine, dans les cellules compétentes.

Des monocouches de cellules primaires de fibroblastes d'embryons de poulets (CEF) sont infectées à 33°C avec VV-Copenhague ts7 (0,1 ufp/cellule) et transfectées avec un coprécipité au phosphate de calcium de l'ADN du virus de type sauvage VV-Copenhague (50 ng/10⁶ cellules) et le plasmide recombinant (50 ng/10⁶ cellules).

Après incubation pendant 2 heures à une température de 33°C, les cellules sont incubées pendant 48 heures à 39,5°C, température qui ne permet pas le développement du virus ts. Des dilutions de virus ts⁺ sont utilisées pour infecter des monocouches de cellules humaines 143B-TK⁻ à 37°C en présence de 5BUDR (150 $\mu$g/ml). Différentes plages de virus TK⁻ sont obtenues à partir de ces cellules qui ont reçu le plasmide recombinant, tandis que les cultures contrôles sans plasmide ne montrent pas de plages visibles. Les virus TK⁻ sont ensuite sous-clonés par une deuxième sélection en présence de 5BUDR.

Une double recombinaison réciproque entre le plasmide pTG2101 ou pTG2102 et le génome de VV aboutit à l'échange du gène TK portant l'insert avec le gène TK du virus, les recombinants devenant ainsi TK⁻.

Les ADN purifiés à partir des différents virus recombinants TK⁻ sont digérés par HindIII et soumis à une électrophorèse sur gel d'agarose. Les fragments d'ADN sont transférés sur un filtre de nitrocellulose selon la technique décrite par Southern (1975). Le filtre est ensuite incubé avec le plasmide pTG2101 ou pTG2102 préalablement marqué avec l'isotope ³²P. Le filtre est lavé et autoradiographié. Après le développement, on observe sur le film la présence de fragments dont la taille atteste du transfert du gène codant pour la p25 dans le génome de la vaccine.

Pour chacun des plasmides pTG2101 et pTG2102, un virus recombinant a été sélectionné et respectivement dénomé VV.TG.25MG et VV.TG.25MA.

Exemple 6 Synthèse de la protéine p25 dans les cellules infectées par les virus de la vaccine recombinants.

La procédure décrite ci-dessous s'applique indifféremment aux virus recombinants VV.TG.25MA ou VV.TG.25MG.

Une monocouche semi-confluente de cellules BHK21 est infectée à une multiplicité de 0,2 ufp/cellule. L'infection est réalisée avec le virus vaccine recombinant dans un milieu de culture G-MEM supplémenté avec 5 % de sérum de veau foetal. Après une incubation à 37°C pendant 18 heures, le milieu de culture est substitué par un milieu renfermant de la méthionine marquée par l'isotope radioactif ³⁵S. En règle générale on ajoute 10 $\mu$l de ³⁵S méthionine (5 mCi/300 $\mu$l) à 1 ml de milieu de culture pour obtenir un marquage efficace des protéines. Après une nouvelle incubation à 37°C le surnageant de culture est séparé des cellules par centrifugation. Les deux fractions sont incubées séparément avec du sérum d'un malade atteint de SIDA, puis avec de la protéine A sépharose. La fraction des immunogllobulines adsorbée sur la protéine A sépharose est alors séparée par centrifugation et ce matérie est soumis à une électrophorèse en gel de polyacrylamide-SDS suivi d'une autoradiographie.

La figure 3 présente les résultats obtenus avec les virus recombinants VV.TG.25MG et VV.TG.25MA. Sur la figure 3A qui représente les résultats obtenus avec les culots cellulaires les pistes 1, 2, 3, 4, 5 correspondent au virus recombinant VV.TG.25MA (2,4, 6, 10 et 24 heures après le marquage à la $^{35}$S Met) ; les pistes 6,7, 8, 9, 10 correspondent au virus recombinant VV.TG.25MG (2, 4, 6, 10 et 24 heures après le marquage) ; la piste M correspond à un marqueur de poids moléculaires (en daltons). La figure 3B représente les résultats obtenus avec les surnageants de culture correspondants.

Ces résultats révèlent clairement qu'une protéine d'un poids moléculaire d'environ 25000 daltons a été immunoprécipitée par le sérum d'un malade atteint de SIDA. Cette protéine est présente dans les cellules BHK infectées par les virus vaccine recombinants, mais absente lors d'une infection par le virus vaccine sauvage (résultat non présenté sur la figure). Les virus vaccine recombinants VV.TG.25MG et VV.TG.25MA assurent donc la synthèse d'une protéine de structure semblable à celle de la p25 du HIV puisqu'elle est parfaitement reconnue par le sérum de malade.

Exemple 7 Production d'anticorps anti-p25 chez des souris vaccinées avec les virus vaccine recombinants VV.TG.25MA et VV.TG.25MG.

4 souris C57/Bl mâles de 7 semaines ont été inoculées par scarification de la base de la queue avec $4 \times 10^7$ ufp pour chacun des virus vaccine recombinants VV.TG.25MG et VV.TG.25MA. Après 4 semaines un prélèvement sanguin a été effectué sur les souris et une deuxième inoculation a eu lieu comme décrit précédemment. Trois semaines plus tard, les animaux sont sacrifiés, et la totalité de leur sang est prélevée par ponction cardiaque.

La capacité de ces sérums à reconnaître les protéines du HIV a été évaluée par la technique d'"immunoblot". Cette technique repose sur l'incubation des sérums avec une feuille de nitro-cellulose sur laquelle les protéines du HIV ont été préalablement adsorbées après électrophorèse en gel de polyacrylamide SDS.

Les résultats obtenus avec les sérums du premier prélèvement, soit un mois après les vaccinations, révèlent que les sérums des souris inoculées avec les virus vaccine recombinants contiennent des anticorps capables de reconnaître spécifiquement la protéine p25 du HIV. De plus, certains sérums sont également capables de reconnaître les épitopes communs entre la p25 et son précurseur p40 comme l'atteste la présence d'une bande au niveau de la p40. Ces anticorps ne sont pas présents dans le sérum de souris vaccinées avec un virus de la vaccine sauvage (résultats non présentés dans la figure).

La figure 4 présente les résultats obtenus avec les sérums prélevés 25 jours après l'injection de rappel. Les pistes 1, 2, 3, 4 correspondent au sérum de 4 souris vaccinées avec le virus recombinant VV.TG.25MA, les pistes 5, 6 correspondent au sérum de 2 souris vaccinées avec le virus recombinant VV.TG.25MG. Les sérums des 2 souris vaccinées avec le virus vaccine recombinant VV.TG.25MG qui ne sont pas présentés dans la figure 4 donnent des résultats similaires. La piste T correspond à un sérum de malade atteint de SIDA.

La présence sur la figure 4 de bandes correspondant à la p25, au précurseur p40 et au doublet caractéristique du précurseur p55 démontre une forte réponse immunologique chez les souris vaccinées avec les virus vaccine recombinants VV.TG.25MA et VV.TG.25MG.

Les résultats présentés dans la figure 4 démontrent clairement que la vaccination de souris avec les recombinants VV.TG.25MA et VV.TG.25MG qui produisent la protéine p25 du HIV induit une réponse immunitaire spécifique chez ces animaux. De plus, les anticorps synthétisés reconnaissent également les précurseurs p55 et p40 ce qui confirme la haute spécificité de la réponse immunitaire chez les souris vaccinées.

La protéine recombinante ainsi obtenue peut être utilisée dans des kits de diagnostic pour détecter les anticorps potentiels présents dans le sang des malades ayant été en contact avec le virus. Ces tests peuvent être mis en oeuvre selon des processus connus de l'homme de l'art, par exemple par ELISA, RIPA, "Westhern Blot" (Immuno-empreinte).

Cette protéine peut également être utilisée pour la réalisation d'hybridomes et d'anticorps monoclonaux destinés à détecter la présence de virus dans des échantillons.

Les plasmides suivants ont été déposés le 28 novembre 1986 à la Collection Nationale de Cultures de Microorganismes de l'INSTITUT PASTEUR, 28 rue du Docteur-Roux, 75724 PARIS CEDEX 15 :

E. coli 1106 pTG2101 sous le n° I-631
E. coli 1106 pTG2102 sous le n° I-632.

Exemple 8 Immunoprécipitation de la protéine p25 du HIV-1 par des anticorps de souris inoculées avec le virus de la vaccine recombinant VV.TG.25MA.

Une préparation de virus HIV-1 dont les protéines avaient été radiomarquées par incorporation de cystéine $^{35}$S a été utilisée comme source d'antigènes. Ces derniers ont été immunoprécipités par du sérum de souris C57/bl inoculées avec le virus de la vaccine recombinant selon le protocole décrit dans l'exemple 7.

50 $\mu$l de préparation virale ont été incubés avec 10 $\mu$l de sérum dans du tampon RIPA pendant une nuit à 4°C, puis mis en présence de protéine A sépharose. La fraction des immunoglobulines adsorbée sur cette matrice est alors séparée du milieu réactionnel par centrifugation. Les complexes antigènes-anticorps sont soumis à une électrophorèse en gel de polyacrylamide-SDS suivie d'une fluorographie.

La figure 5 présente les résultats obtenus. Les pistes a, b et c correspondent respectivement a des immunoprécipitations réalisées avec le sérum de souris et deux sérums humains différents. L'autoradiographie révèle que la protéine p25 est la seule protéine du HIV-1 immunoprécipitée par le sérum de souris inoculée avec le virus de la vaccine recombinant VV.TG.25MA (sa position est marquée d'une flèche).

Ces anticorps de souris sont donc capables de reconnaître non seulement la p25 fixée sur la nitrocellulose (lors de l'analyse par la technique de Western blot - voir l'exemple 7) mais aussi la p25 native telle qu'on la trouve dans la particule virale.

Cette propriété démontre l'intérêt de ce virus recombinant VV.TG.25MA puisqu'il provoque, chez la souris, l'apparition d'anticorps capables de reconnaître les épitopes séquentiels et conformationnels de la protéine p25 du HIV-1.

Exemple 9 Reconstruction du gène gag complet et insertion dans le virus de la vaccine.

Le phage M13TG1120 contient le fragment HindIII-EcoRV du plasmide pJ19-13 (nucléotides 1258 à 2523) et code pour l'extrémité C-terminale de la p25 et toute la p13. Ce phage a la structure suivante :

Le reste du gène gag est contenu dans les 2 plasmides pJ19-1 et pJ19-17 (fragments HindIII).

La première étape consiste à ajouter la partie codant pour la p18 en amont du fragment HindIII-EcoRV de M13TG1120.

Le plasmide pJ19-1 contient les séquences du génome du virus HIV-1$_{BRU}$ comprise entre les nucléotides 77 et 631 dans le plasmide pUC18 :

Le sens de transcription de l'ADN va de SstI vers AccI, et le codon correspondant à l'ATG initiateur de gag est situé en position 335 (Wain-Hobson, 1985).

Le fragment SstI-HindIII est donc excisé du plasmide pJ19-1 et inséré entre les sites BamHI et HindIII du bactériophage M13TG1120 à l'aide de l'adaptateur 5′-GATCAGCT-3′ pour générer le M13TG191, dont la structure est la suivante :

L'étape suivante consiste en l'insertion du fragment central HindIII (nucléotides 631-1258) du plasmide pJ19-17 dans le site HindIII de M13TG191. Ceci génère le bactériophage M13TG192 :

Le fragment SalI-EcoRI de M13TG192 est ensuite inséré entre les sites SalI et EcoRI du plasmide pTG186POLY pour générer le plasmide pTG1144, où la séquence codante du gène gag est placée sous le contrôle du promoteur 7.5 K du virus de la vaccine. Ce plasmide permet l'intégration, par recombinaison, du bloc d'expression de gag dans le virus de la vaccine.

Exemple 10 Introduction d'un codon stop à la fin du cadre de lecture de la p18.

La p18 se termine aux acides aminés Glu Asn Tyr. Il s'agit donc d'introduire un codon de terminaison au niveau de cette séquence. Pour ce faire, une mutagénèse localisée est effectuée sur le M13TG192 avec l'oligonucléotide :
5′-GCACTATAGACTAGTTTTGGCTG-3′
pour générer la séquence suivante (M13TG1154) :

$$\overrightarrow{\text{p25}}$$

Val Ser Gln Asn Tyr Pro Ile Val Gln
GTC AGC CAA AAT TAC CCT ATA GTG CAG
        *       *   *
  C AGC CAA AAC TAG TCT ATA GTG C
          Spe I
  Glu Asn Stop Pro

Le fragment SalI-SphI du bactériophage M13TG1154 est ensuite cloné entre les sites SalI et SphI du plasmide pTG186POLY pour générer le plasmide pTG1197. Dans ce plasmide, le cadre de lecture de la p18 est placé sous le contrôle du promoteur 7.5 K du virus de la vaccine. Ce plasmide permet l'intégration, par recombinaison, du bloc d'expression de la p18 dans le virus de la vaccine.

Exemple 11 Construction d'un plasmide permettant l'expression d'une p18 fusionnée en sa partie N-terminale avec le peptide hydrophobe N-terminal du gène HA du virus de la rougeole.

La protéine p18 est myristilée in vivo. Elle peut donc être ancrée dans la membrane cytoplasmique par l'intermédiaire de cet acide gras. Pour améliorer la présentation de cet antigène, on peut remplacer cet ancrage par un peptide hydrophobe, comme celui du gène de l'hémagglutinine (HA) du virus de la rougeole par exemple.

Le plasmide pTG2155 (brevet français 87.12396) contient la séquence codante du peptide N-terminal hydrophobe du gène HA, en aval du promoteur 7.5 K du virus de la vaccine. Un site BamHI est situé après la séquence de la zone hydrophobe et permet l'intégration d'un ADN étranger.

On a introduit un site BglII en amont de l'ATG initiateur de la p18 afin de permettre l'intégration du gène dans le site BamHI du plasmide pTG2155. Ceci est réalisé grâce à l'oligonucléotide 5′-CTCGCACC-CATAGATCTCCTTCTAG-3′ en mutant le phage M13TG1154 :

```
                                    ──→ p18
    5'  ...  GGCTAGAAGGAGAGAG(ATG)GGTGCGAGA... 3'

    3'          GATCTTCCTCTAGATACCCACGCTC      5'

                        BglII
```

Le phage obtenu est appelé M13TG2161.

L'ADN de ce dernier est ensuite excisé par BglII et inséré dans le site BamHI du plasmide pTG2155 pour générer le plasmide pTG2160, qui permet le transfert dans le génome du virus de la vaccine. Le virus recombinant correspondant sera appelé VV.TG.2161.

Exemple 12 Expression des protéines gag p55 et p18 dans le virus de la vaccine.

Les virus recombinants de la vaccine portant les inserts décrits dans les exemples précédents, VV.TG.1144 et 1197 codent respectivement pour les protéines p55 et p18 du virus HIV-1$_{BRU}$. Ces virus ont été utilisés pour infecter des tapis cellulaires, comme décrit dans l'exemple 6.

Par immunoprécipitation, on peut mettre en évidence la production des 2 protéines qui migrent après électrophorèse sur gel de polyacrylamide SDS-PAGE avec la mobilité attendue, et dont les extrémités N-terminales sont myristilées. Le virus VV.TG.2161 permet la synthèse d'une protéine non myristilée de 23 kDa comme attendu. Une autre forme de la p18 migrant avec une mobilité apparente de 30 kDa peut être observée sur l'autoradiographie. Cette bande ne correspond pas à une forme glycosylée de la p18.

Une expérience d'immunofluorescence sur cellules de souris LTK⁻ non fixées permet de visualiser la p18 produite par les virus VV.TG.1197 et 2161 à la surface des cellules infectées.

REFERENCES     Drillien, R. & Spehner, D., Virology 131, 385-393 (1983).

Fisher, A.G., Feinberg, M.B., Josephs, S.F., Harper, M.E., Marselle, L.M., Reyes, G., Gouda, M.A., Aldovini, A., Debouk, C., Gallo, R.C. & Wong-Staal, F. Nature 320, 367-371 (1986).

Kieny, M.P., Lathe, R. & Lecocq, J.P. Gene 26, 91-99 (1983).

Kozak, M. Cell 44, 283-292 (1986).

Lathe, R., Hirth, P., Dewilde, M., Harford, N & Lecocq, J.P. Nature 284, 473-474 (1980).

Mackett, M., Smith, G.L. & Moss, B. Proc. Natl. Acad. Sci. USA. 79, 7415-7419 (1982).

Mackett, M. & Smith, G.L. J. Gen. Virol. 67, 2067-2082 (1986).

Messing & Vieras, Gene 19, 269-276 (1982).

Moss, B. Banbury Report Apr-May 1985 Cold Spring Harbor Laboratory, CSH, NY, USA.

Muesing, M.A., Smith, D.H., Cabradilla, C.D., Benton, C.V., Lasky, L.A. & Capon, D.J. Nature 313, 450-458 (1985).

Panicali, D. & Paoletti, E. Proc. Natl. Acad. Sci. USA 79, 4927-4931 (1982).

Panicali, D., Davis, S.W., Weinberg, R.L., Paoletti, E. Proc. Natl. Acad. Sci. USA 80, 5364-5368 (1983).

Ratner, L., Haseltine, W., Patarca, R., Livak, K.J., Starcich, B., Josephs, S.F., Doran, E.R., Rafalski, J.A., Whitehorn, E.A., Baumeister, K., Ivanoff, L., Petterway Jr., S.R., Pearson, M.L., Lautenberger, J.A., Papas, T.S., Ghrayeb, J., Chang, N.T., Gallo, R.C. & Wong-Staal, F. Nature 313, 277-284 (1985).

Rosen, G.A., Sodroski, J.G., Goh, W.C., Dayton, A.I., Lippke, J. & Haseltine, W. Nature 319, 555-559

EP 0 276 591 B1

(1986).

Smith, G.L., Mackett, M., Moss, V. Nature 302, 490-495 (1983).

Smith, G.L., Murphy, B.R., Moss, B. Proc. Natl. Acad. Sci. USA 80, 7155-7159 (1983). Starcich, B.R., Hahn, B.H., Shaw, G.M., McNeely, P.D.,

Modrow, S., Wolf, H., Parks, E.S., Parks, W.P., Josephs, S.F., Gallo, R.C. & Wong-Staal, F. Cell 45, 637-648 (1986).

Wain-Hobson, S., Sonigo, P., Danos, O., Cole, S. & Alizon, M. Cell 40, 9-17 (1985).

**Revendications**

1. Vaccin contre le virus HIV responsable du SIDA, caractérisé en ce qu'il est constitué par un vecteur viral qui comporte au moins :
   - une partie du génome d'un virus vecteur,
   - le gène gag complet ou un de ses sous-fragments, notamment un gène codant pour la protéine p25 ou un gène codant pour la protéine p18 du virus HIV, responsable du SIDA,
   - ainsi que les éléments assurant l'expression de cette protéine dans les cellules eucaryotes en culture.

2. Vaccin selon la revendication 1, caractérisé en ce que la partie du génome d'un virus est une partie du génome de poxvirus.

3. Vaccin selon la revendication 2, caractérisé en ce que le poxvirus est le virus de la vaccine.

4. Vaccin selon l'une des revendications 1 à 3, caractérise en ce que la séquence d'ADN codante est sous la dépendance d'un promoteur d'un gène du poxvirus.

5. Vaccin selon la revendication 4, caractérisé en ce que le promoteur est un promoteur du gène de la vaccine.

6. Vaccin selon la revendication 5, caractérisé en ce que la séquence d'ADN codante est sous le contrôle du promoteur du gène de la protéine 7,5 K de la vaccine.

7. Vaccin selon l'une des revendications 4 à 6, caractérisé en ce que la séquence codante est clonée dans le gène TK de la vaccine.

8. Vaccin selon l'une des revendications précédentes, caractérisé en ce qu'il comporte au moins :
   - une partie du génome d'un virus vecteur,
   - un gène codant pour la protéine p25 du virus HIV responsable du SIDA,
   - ainsi que les éléments assurant l'expression de cette protéine dans les cellules eucaryotes en culture.

9. Vaccin selon la revendication 8, caractérisé en ce qu'il est choisi parmi : VV.TG.25MG-VV.TG.25MA, obtenu à partir des plasmides pTG 2101 et pTG2102 déposés à la CNCM sous le numéro I-631 et I-632.

10. Vaccin selon l'une des revendications 1 à 7, caractérisé en ce qu'il comporte au moins :
    - une partie du génome d'un virus vecteur,
    - un gène codant pour la protéine p18 du virus HIV, responsable du SIDA,
    - ainsi que les éléments assurant l'expression de cette protéine dans les cellules eucaryotes en culture.

**Claims**

1. A vaccine against HIV virus responsible for AIDS characterized in that it comprises a viral vector, which contains at least :
   - a portion of the genome of a vector virus,
   - the complete gag gene or one of its subfragments, in particular a gene coding for the p25 protein or a gene coding for the p18 protein of the HIV virus, responsible for AIDS,

- as well as the elements which provide for the expression of this protein in eukaryotic cells in culture.

2. Vaccine as claimed in claim 1, which the portion of the genome of a virus is a portion of the pox virus genome.

3. Vaccine as claimed in claim 2, in which the pox virus is vaccinia virus.

4. Vaccine as claimed in one of claims 1 to 3, in which the DNA coding sequence is under the control of a promoter of a pox virus gene.

5. Vaccine as claimed in claim 4, in which the promoter is a promoter of the vaccinia gene.

6. Vaccine as claimed in claim 5, in which the DNA coding sequence is under the control of the promoter of the gene for the 7.5 K protein of vaccinia.

7. Vaccine as claimed in one of claims 4 to 6, in which the coding sequence is cloned into the TK gene of vaccinia.

8. Vaccine as claimed in one of the preceding claims, which contains at least :
   - a portion of the genome of a vector virus,
   - a gene coding for the p25 protein of the HIV virus, responsible for AIDS,
   - as well as the elements which provide for the expression of this protein in eukaryotic cells in culture.

9. Vaccine as claimed in claim 8, which is chosen from : VV.TG.25MG and VV.TG.25MA.

10. Vaccine as claimed in one of claims 1 to 7, which contains at least :
    - a portion of the genome of a vector virus,
    - a gene coding for the p18 of the HIV virus, responsible for AIDS,
    - as well as the elements which provide for the expression of this protein in eukaryotic cells in culture.

**Patentansprüche**

1. Impfstoff gegen das HIV-Virus, das für AIDS verantwortlich ist, dadurch gekennzeichnet, daß er besteht aus einem Virus-Vektor, der mindestens enthält:
   - einen Teil des Genoms eines Virus-Vektors,
   - das vollständige gag-Gen oder eines seiner Unterfragmente, insbesondere ein Gen, das für das p25-Protein des für AIDS verantwortlichen HIV-Virus codiert, oder ein Gen, das für das p18-Protein des für AIDS verantwortlichen HIV-Virus codiert, sowie
   - die Elemente, welche die Expression dieses Proteins in die Eukariotenzellen in der Kultur gewährleisten.

2. Impfstoff nach Anspruch 1, dadurch gekennzeichnet, daß der Teil des Genoms eines Virus ein Teil des Genoms des Poxvirus (Pockenvirus) ist.

3. Impfstoff nach Anspruch 2, dadurch gekennzeichnet, daß es sich bei dem Poxvirus (Pockenvirus) um das Vaccine-Virus handelt.

4. Impfstoff nach einem der Ansprüche 1 bis 3, dadurch gekennzeichnet, daß die codierende DNA-Sequenz kontrolliert wird von einem Promotor eines Gens des Poxvirus (Pockenvirus).

5. Impfstoff nach Anspruch 4, dadurch gekennzeichnet, daß es sich bei dem Promotor um einen Promotor des Vaccine-Gens handelt.

6. Impfstoff nach Anspruch 5, dadurch gekennzeichnet, daß die codierende DNA-Sequenz unter der Kontrolle des Promotors des Vaccine-Gens des Proteins 7,5 K steht.

**7.** Impfstoff nach einem der Ansprüche 4 bis 6, dadurch gekennzeichnet, daß die codierende Sequenz in dem Vaccine Gen TK kloniert ist.

**8.** Impfstoff nach einem der vorhergehenden Ansprüche, dadurch gekennzeichnet, daß er mindestens enthält:
- einen Teil des Genoms eines Virus-Vektors,
- ein Gen, das für das p25-Protein des für AIDS verantwortlichen HIV-Virus codiert, sowie
- die Elemente, welche die Expression dieses Proteins in die Eukariotenzellen in der Kultur gewährleisten.

**9.** Impfstoff nach Anspruch 8, dadurch gekennzeichnet, daß er ausgewählt wird aus der Gruppe VV.TG.25MG-VV.TG.25MA, erhalten aus den Plasmiden pTG2101 und pTG2102, die bei CNCM unter der Nr. I-631 und I-632 hinterlegt sind.

**10.** Impfstoff nach einem der Ansprüche 1 bis 7, dadurch gekennzeichnet, daß er mindestens enthält:
- einen Teil des Genoms eines Virus-Vektor
- ein Gen, das für das p18-Protein des für AIDS verantwortlichen HIV-Virus codiert, sowie
- die Elemente, welche die Expression dieses Proteins in die Eukariotenzellen in der Kultur gewährleisten.

FIG_1

CONSTRUCTION DU PLASMIDE M13 TG 1124

FRAGMENT P2-R1
CLONE DANS LE pTG186 poly

CONSTRUCTION DU PLASMIDE M13TG1125

FIG_2

EXPRESSION DE LA PROTEINE P25 PAR LES VIRUS RECOMBINANTS

FIG. 3A

FIG. 3B

## DETECTION D'ANTICORPS CONTRE LA P 25

FIG_4

FIG. 5